# EUROPEAN PATENT APPLICATION

(11) **EP 2 036 884 A1**
(43) Date of publication of application: **18.03.2009**
(21) Application number: 07380251.4
(22) Date of filing: 14.09.2007
(51) Int. Cl.: C07C 269/04, C07C 271/24, C07C 271/28, C07C 263/04, C07C 265/10, C07C 265/14

(54) **Process for producing fluorinated isocyanates and carbamates**

(71) Applicant: REPSOL YPF S.A., 28046 Madrid (ES)
(72) Inventor: Padilla Polo, Ana, 28903 Getafe, Madrid (ES); Ruiz Santa Quiteria, Valentin, 28039 Madrid (ES); Corma, Canós, Avelino, 46020 Valencia (ES); Garcia Gómes, Hermenegildo, 46410 Sueca Valencia (ES); Juárez Marín, Raquel, 46910 Benetúser Valencia (ES)
(74) Representative: ABG Patentes, S.L.

(57) **Abstract**

The invention relates to a process for producing fluorinated carbamates comprising the reaction between an amine or polyamine and a fluorinated carbonate, in the presence of a catalyst which does not contain metals and comprises at least one tertiary amine group. The process can also comprise an additional step in which the carbamates are transformed into the corresponding isocyanates.

## Description

### Field of the Invention

The present invention relates to a process for producing se fluorinated isocyanates and carbamates by means of reacting amines or polyamines with fluorinated carbonates in the presence of catalysts.

### State of the Art

Carbamates or urethanes are organic compounds formally derived from carbonic acid which contain a carbonyl group covalently bonded simultaneously to an alkoxy group an amino group.

One of the most common routes among the possible alternative routes for synthesizing carbamates consists of reacting organic carbonates with amines (Scheme 1).

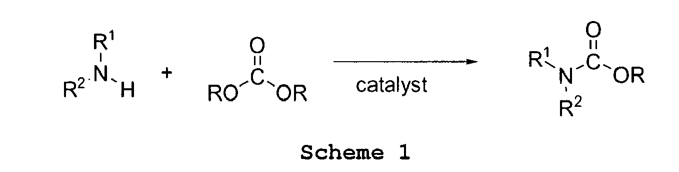

However, dialkyl carbonates can react with amines in two different ways, either forming carbamates or as alkylating agents. This second reaction acts by competing with the first and is unwanted when the synthesis of carbamates is desired (Scheme 2).

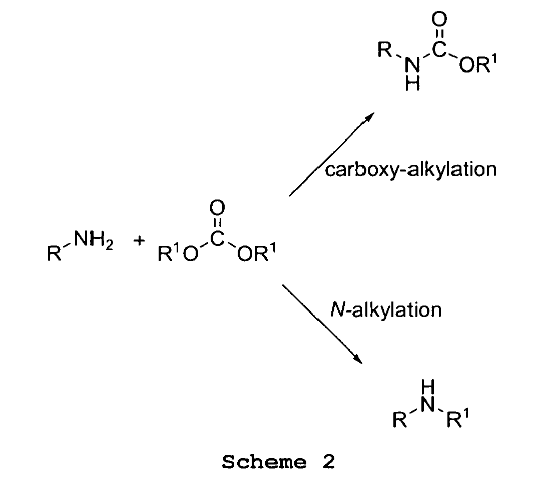

The most widely used organic carbonate which has been the object of the greatest attention due to its availability is dimethyl carbonate, although other dialkyl carbonates and alicyclic carbonates can act in a completely similar manner to dimethyl carbonate.

Patent US-A 5,347,034 describes a process for producing poly(O-alkylurethanes) of the diphenyl methane series by means of reacting the corresponding amines with dialkyl carbonates in the presence of a metal catalyst, such that poly(O-alkylurethanes) crystallized in a pure form by cooling.

EP-A 520,273 describes the production of bis(ethoxycarbonylamino)toluene derived from toluenediamine, in the presence of catalysts based on Sn, Zn or Pb.

Patent EP-A 391,473 describes a process for producing carbamates with reduced amounts of catalyst, first reacting an amine with a cyclo(alkyl carbonate) to obtain mixtures of carbamates and urea and then reacting the urea with carbonate to produce the corresponding carbamate, finally recovering the carbamate from the reaction mixture.

Patent DE-A- 3,202,690 describes a method for preparing aromatic urethanes by reacting aromatic amines with alkyl carbonates in the presence of alkali or alkaline earth metal alcoholates.

Patent US-A 4,268,683 describes a method for preparing carbamates by reacting organic carbonates with aromatic amines in the presence of soluble zinc, tin or cobalt salts which are only active at temperatures of 140°C and above.

Patent US-A 4,268,683 proposes the use of soluble zinc or tin salts as catalysts in the reaction mixture.

EP-A 48,371 describe the preparation of N,O-disubstituted urethanes by reacting primary amines with dialkyl carbonates in the presence of catalysts, formed by neutral or basic, inorganic or organic lead, titanium, zinc or zirconium compounds.

The preparation of aliphatic carbamates using sodium methoxide as a catalyst is proposed in US-A 5,138,015 and US-A 5,315,034.

JP 2004244349 describes the preparation of alkyl carbamates by reacting non-aromatic amines with dialkyl carbonates in the presence of europium, gadolinium, terbium, dysprosium, holmium, erbium, thulium and lutetium compounds.

EP 0323514 and EP 0570071 describe a process for preparing aliphatic isocyanates consisting of reacting dimethyl carbonate with an aliphatic diamine in the presence of basic catalysts (alkali and alkaline earth metal alcoholates), followed by the thermal decomposition of the carbamate in the presence of metal catalysts.

EP 0752413 and US 5698731 describe the production of aromatic carbamates by reacting organic carbonates and aromatic amines, using N,N-disubstituted zinc or copper carbamate complexes as a catalyst. EP0752414 proposes using a zinc or copper carbonate hydroxide as a catalyst.

WO 98/55450 describes a process for preparing carbamates in the presence of basic metal catalysts on an inert support.

WO 98/56758 proposes a process for obtaining organic isocyanates, which includes, as a first step, reacting amines and organic carbonates in the presence of a metal salt and an organic solvent.

EP 0881213, WO 01/68590 and JP 2004262892 propose the synthesis of carbamates derived from aromatic amines using Lewis acids as catalysts.

US 5789614 describe the reaction of dimethyl carbonate with aliphatic diamines in the presence of alkali metal alkoxides.

WO 99/47493 describes a method for preparing organic carbamates by reacting amines and heteroatom-substituted alkyl carbonates in the presence of metal catalysts (Ti, Zr, Mn, Fe, Co, Ni, Cu, Zn, Sn, Pb, Bi and Cd).

In WO 01/56977, EP 1255728 and US 2003/162995, the reaction between aromatic amines and organic carbonates is carried out in the presence of a catalyst selected from inorganic and organic salts of metals selected from Zn, Sn, Pb or Cu.

US 5451697A1 describes a process comprising the reaction between carbon dioxide, a primary amine, an aprotic organic solvent and a base selected from a phosphazene, an organic, nitrogenous base and mixtures thereof. Said base is added in stoichiometric proportions or in excess and the reaction product is the ammonium carbamate salt (RNHCO₂-), which forces later adding an anhydride if the corresponding isocyanate is to be formed.

It has also been published that derivatives of 1,5,7-triazabicyclo[4.4.0]dec-5-ene covalently anchored to silicas can promote the carboxy-methylation of aliphatic amines with high yields, although this same material shows a considerably lower efficiency when it is aniline (G. Sartori et al., Journal of Catalysis, 205, 199-204, 2002).

The methods proposed in the current art for producing carbamates by reacting amines and organic carbonates have a series of drawbacks. Firstly, the use of metal catalysts for the mentioned reaction is undesirable because they contaminate the reaction products and their elimination in order to obtain carbamates with the purity required by their subsequent industrial use is difficult and expensive. Furthermore, these catalysts generally lose their activity in the course of the reaction and in the event of being recovered, they cannot be recycled into the process, which involves a relatively high catalyst cost and the formation of unwanted metal waste from the environmental point of view.

Some processes further produce an excessive amount of N-alkylation and/or low yield. Another drawback of some of these processes is the high proportion of impurities.

### Summary of the Invention

One aspect of the present invention is a process for producing fluorinated carbamates comprising the reaction between an amine or polyamine and a fluorinated carbonate of formula (OR)(OR')C=O, wherein R and R' are independently selected from a substituted or non-substituted fluorinated alkyl group with 1 to 20 carbon atoms, in the presence of a catalyst which does not contain metals and comprises at least one tertiary amine group.

The process of the invention allows obtaining fluorinated carbamates with a high yield and high purity. This makes the carbamates obtained according to the method of the present invention especially suitable for their subsequent transformation into the corresponding isocyanates.

The reaction conditions for the formation of carbamates are additionally mild, for example, temperatures between 100 and 250°C, and sufficient pressure so as to maintain the reactants in liquid phase.

### Detailed Description of the Invention

### Amines and Polyamines

The amines which can be used in the context of the present invention are aliphatic or aromatic amines and polyamines.

According to a preferred embodiment, said amines or polyamines have the formula (III)

R₁₄(NH₂)ₙ (III)

wherein R₁₄ is selected from the group consisting of substituted or non-substituted alkyl with 1 to 20 carbon atoms, substituted or non-substituted aryl with 6 to 15 carbon atoms, substituted or non-substituted arylalkyl with 7 to 15 carbon atoms, substituted or non-substituted alkenyl with 2 to 20 carbon atoms, substituted or non-substituted alkynyl with 2 to 20 carbon atoms, substituted or non-substituted cycloalkyl with 3 to 20 carbon atoms, substituted or non-substituted cycloalkenyl with 4 to 20 carbon atoms and substituted or non-substituted cycloalkynyl with 7 to 20 carbon atoms; and n is 1, 2, 3, 4, 5 or 6.

According to another preferred embodiment, said amine is selected from the group consisting of n-propylamine, isopropylamine, n-butylamine, n-hexylamine, n-octylamine, laurylamine, cyclopentylamine, cyclohexylamine, ciclooctylamine, 1,2-diaminoethane, 1,12-diaminododecane, 1,4-diaminocyclohexane, 1,3-bis(aminomethyl)cyclohexane, 1,4-bis(aminomethyl)cyclohexane, hydrogenated 2,4-diaminodiphenylmethane, hydrogenated toluylenediamine, aniline, benzylamine, 2-aminotoluene, 4-aminotoluene, 2,4'-diaminodiphenylmethane, 4,4'-diaminodiphenylmethane, 2,2'-diaminodiphenylmethane, 2,4-toluenediamine, 2,6 toluenediamine, m-phenylenediamine, 1,5 diaminonaphthalene and mixtures thereof.

### Fluorinated Carbonates

The carbonates of the present invention are fluorinated carbonates, i.e., those comprising two identical or different fluorinated alkyl groups. For the purposes of the present invention, "fluorinated alkyl group" is understood as an alkyl group comprising at least one fluorine atom.

According to a preferred embodiment, R and R' are independently selected from the group of linear or branched, substituted or non-substituted fluorinated alkyls with 1, 2, 3, 4, 5, 6, 7, or 8 carbon atoms, preferably 1, 2, 3 or 4 carbon atoms.

Fluorinated carbonates are preferably selected from the group consisting of bis(2-fluoroethyl) carbonate, bis(3-fluoropropyl) carbonate, bis(2,2,2-trifluoroethyl) carbonate, bis(1,3-difluoro-2-propyl) carbonate, bis(1,1,1-trifluoro-2-propyl) carbonate, bis(2,2,3,3-tetrafluoropropyl) carbonate, bis(2,2,3,3,3-pentafluoropropyl) carbonate, bis(1-fluoro-2-butyl) carbonate, bis(2-fluoro-1-butyl) carbonate, bis(1-fluoro-2-methyl-2-propyl) carbonate, bis(2-fluoro-2-methyl-1-propyl) carbonate, bis(1H,1H,2H,2H-perfluoro-1-hexyl) carbonate, bis(perfluorooctyl) carbonate, bis(1,1,1,3,3,3-hexafluoro-2-propyl) carbonate, etc, preferably bis(2,2,2-trifluoroethyl) carbonate.

### Catalysts

As indicated above, the catalysts of the present invention do not contain metals and comprise at least one tertiary amine group.

The pka associated to the amine group of the catalyst is preferably greater than 6, preferably greater than 9, preferably a pka greater than 12, more preferably a pka greater than 14.

For the purposes of the present invention, "tertiary amine" is understood as an amine the nitrogen atom of which is not directly bonded to a hydrogen atom.

The catalysts of the present invention can selectively and efficiently promote the carbamoylation of fluorinated carbonates.

The catalysts of the present invention include compounds containing P-N bonds, especially phosphazenes. Phosphazenes have structures in which there are one, two or several pentavalent phosphorus atoms covalently bonded by means of single bonds to dialkylamino groups and by means of a double bond to an alkylimino group. According to a preferred embodiment, said phosphazene is of formula (I) 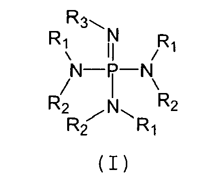 wherein
each R₁ and R₂ is independently selected from the group consisting of a substituted or non-substituted alkyl group with 1 to 20 carbon atoms, a substituted or non-substituted aryl group with 6 to 15 carbon atoms, a substituted or non-substituted arylalkyl group with 7 to 15 carbon atoms and a substituted or non-substituted alkylaryl group with 7 to 15 carbon atoms;
and wherein at least one R₁ or R₂ group can form together with another R₁ or R₂ group a substituted or non-substituted alkylidene group with 1 to 20 carbon atoms; or
R₁ and R₂ together form a group of formula (II) 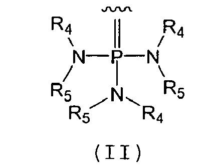 wherein each R₄ and R₅ is independently selected from the group consisting of a substituted or non-substituted alkyl group with 1 to 20 carbon atoms, a substituted or non-substituted aryl group with 6 to 15 carbon atoms, a substituted or non-substituted arylalkyl group with 7 to 15 carbon atoms and a substituted or non-substituted alkylaryl group with 7 to 15 carbon atoms;
   and wherein at least one R₄ or R₅ group can form together with another R₄ or R₅ group a substituted or non-substituted alkylidene group with 1 to 20 carbon atoms; or R₄ and R₅ together form a group of formula (II); and
   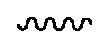 indicates where the group of formula (II) is bonded to the rest of the phosphazene; and
   R₃ is selected from the group consisting of a substituted or non-substituted alkyl group with 1 to 20 carbon atoms, a substituted or non-substituted aryl group with 6 to 15 carbon atoms, a substituted or non-substituted arylalkyl group with 7 to 15 carbon atoms and a alkylaryl group with 7 to 15 carbon atoms;
   the total number of phosphorus atoms being comprised between 1 and 40
and salts thereof.

For example, when R₃ is a terbutyl group and each of R₁ and R₂ is methyl, the phosphazene group comprises a single phosphorus atom, and the compound of formula (I) is

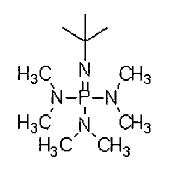

In another embodiment, R₃ is terbutyl and each R₁ and R₂ together form a radical of formula (II), wherein each R₄ and R₅ are methyl, resulting in a phosphazene of formula

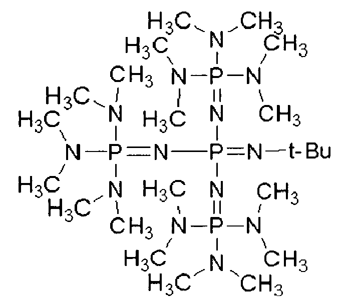

According to an embodiment of the invention, the phosphazene comprises 1 phosphorus atom, i.e., none of R₁ and R₂ together form a radical of formula (II).

According to another preferred embodiment, the phosphazene comprises from 2 to 20 phosphorus atoms, preferably from 2 to 10, more preferably from 2 to 5.

In some phosphazenes, one or more phosphorus atoms can be found forming a salt, preferably with halide ions, for example, the phosphazenes

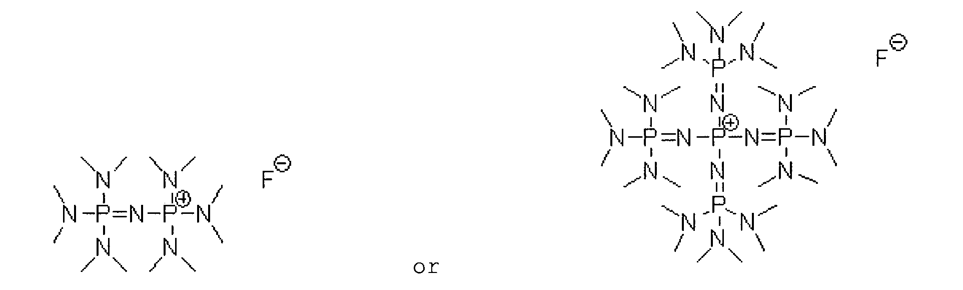

In this case, the pentavalent phosphorus atom is bonded by means of a single bond to four dialkylamino groups.

The phosphazene is preferably selected from the group consisting of

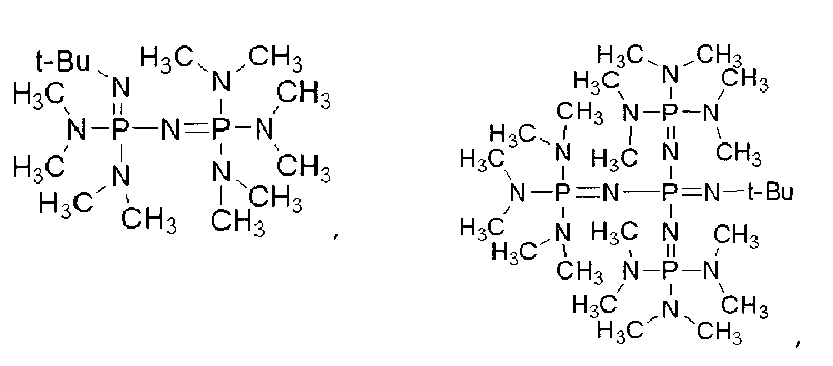

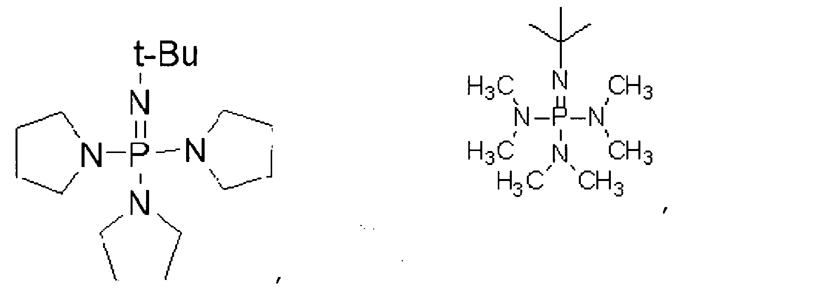

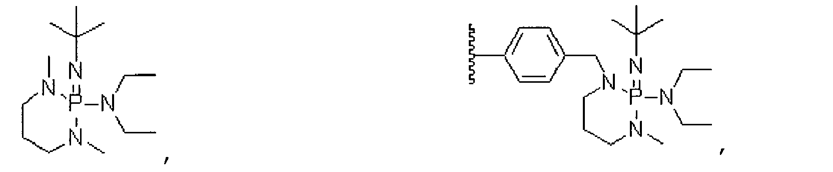

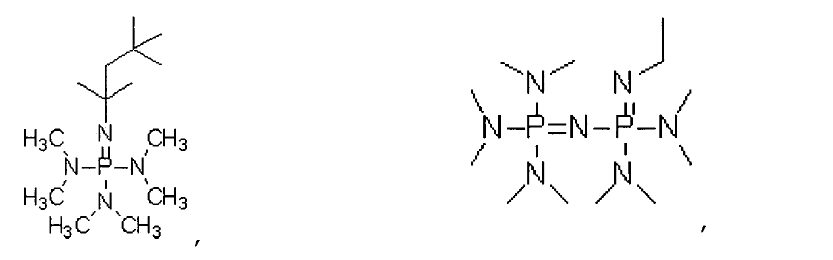

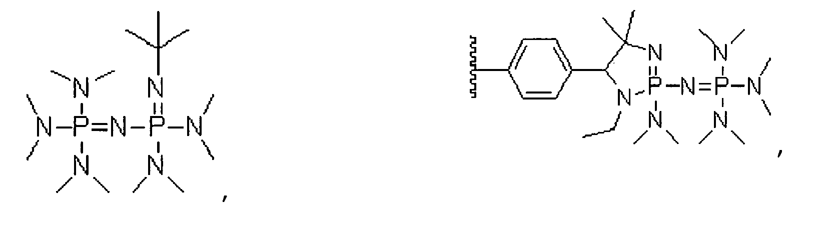

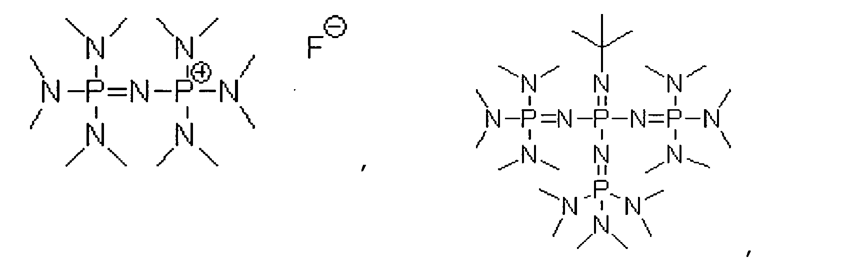

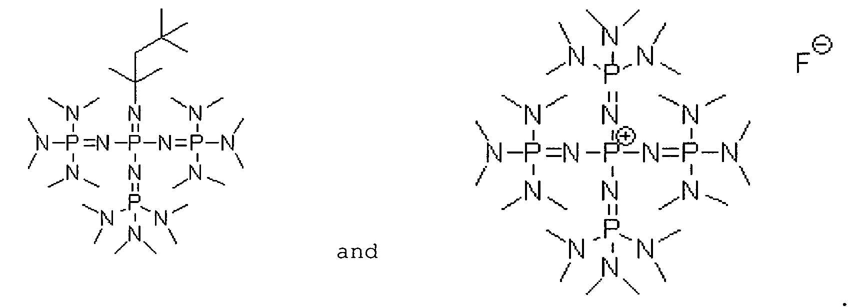

The catalyst of the present invention can alternatively be a cyclic amine of formula (IV) 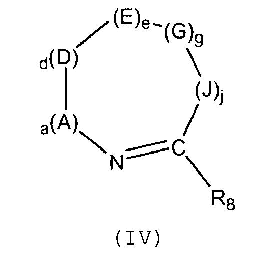 wherein each of a, d, e, g and j is 0 or 1, the total sum of a, d, e, g and j being equal to 3, 4 or 5;
each of the ring members A, D, E, G and J is independently selected from the group consisting of - C(H)=, -C(R₇)=, -N=, -N(R₇)-, -S-, and -O-, the members -N=, -N(R₇)-, -S-, and -O- not being able to be in positions contiguous to one another, and the sum of -N=, -N(R₇)-, -S-, and -O- being equal to 0, 1 or 2;
wherein R₇ is selected from the group consisting of substituted or non-substituted alkyl of 1 to 20 carbon atoms and dialkylamino of formula -N(R₉)(R₁₀),
wherein R₈ and R₁₀ are independently selected from a substituted or non-substituted alkyl group with 1 to 20 carbon atoms; and
R₈ is selected from the group consisting of hydrogen, substituted or non-substituted alkyl of 1 to 20 carbon atoms and a dialkylamino group of formula - N(R₉)(R₁₀), wherein R₉ and R₁₀ are independently selected from a substituted or non-substituted alkyl group with 1 to 20 carbon atoms; or
- an amine of formula (V) 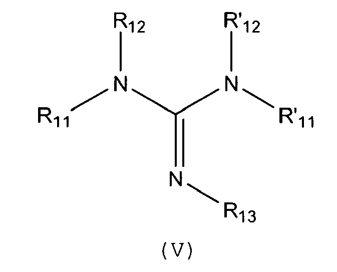 wherein each R₁₁, R₁₂, R₁₃, R'₁₁ and R'₁₂ group is independently selected from the group consisting of substituted or non-substituted alkyl with 1 to 20 carbon atoms and substituted or non-substituted cycloalkyl with 3 to 20 carbon atoms; and wherein at least one R₁₁, R₁₂, R₁₃, R'₁₁ or R'₁₂ group can form together with another R₁₁, R₁₂, R₁₃, R'₁₁ or R'₁₂ group a substituted or non-substituted alkylidene group with 1 to 20 carbon atoms.

The cyclic amine of formula (IV) is preferably a pyridine of formula (IV') 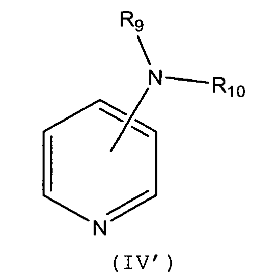 wherein R₉ and R₁₀ are as defined above. R₉ and R₁₀ are preferably methyl.

According to a preferred embodiment, the catalyst is selected from the group consisting of imidazole, thiazole and guanidine derivatives such as 1,5,7-triazabicyclo[4.4.0]-5-decene, 1,6,8-triazabicyclo[5.4.0]-6-undecene or 2,11-diazabicyclo[5.4.0]-1-undecene, for example.

None of these described catalysts contains metals, which is advantageous from the environmental point of view.

### Solid Supports

The catalysts of the present invention can act as catalysts in solution, but can further comprise a solid support, preferably with a large surface area.

The support is preferably an inorganic support which is selected from the group consisting of amorphous silicas, structured mesoporous silicas such as MCM-41, MCM-48, SBA-15, FDU, mixed silicon and alumina oxides, laminar or fibrous clays, crystalline or amorphous aluminosilicates and zeolites, metal oxides wherein the particle size is submicrometric such as cerium oxide and other rare earth oxides, iron oxide, hydrotalcites, mixed metal oxides and magnesium oxides.

The inorganic solids which can be used as a support include any type of silicas including amorphous silicas, porous silicas with a regular or irregular pore distribution with diameters from subnanometric to tens of nanometers and a mono-, bi- or tridirectional pore geometry. Ordered or non-ordered mesoporous silicas, as well as meso/macroporous silicas are included in these silicas acting as a support. Ordered or non-ordered, amorphous aluminas or aluminas in any crystallographic phase and mixed oxides such as silica-alumina, silica-titania, alumina-phosphorus oxide for example, can also be used as supports. Other doped or undoped metal oxides, such CeO₂, WO₃, TiO₂, FeₓO_{y}, RuO₂, VₓO_{y}, MoO₃, etc., with or without altered stoichiometry, prepared such that the surface area and their spatial structure is controlled, can also be suitable supports for the present invention. Sulfides, selenides and other metal chalcogenide can also be supports for the present invention.

When the catalyst comprises a support, the interaction formed between the support and the rest of the catalyst can be of any nature, including Coulomb forces of attraction, hydrogen bonds and van der Waals forces.

The catalysts can also comprise polymeric supports, preferably organic polymers. Said polymers can be natural or synthetic and can be soluble or insoluble in the reaction medium. The polymers include derivatives of glucosamines (chitin and chitosan), polystyrenes, acrylates, polyethylene glycol, polyethoxylates, etc. Polymers with a dendrimeric structure, which are especially advantageous in the case of phosphazenes, and particularly polyamidoamine (PAMAM) derivatives, are also included.

The average weights of the polymers and polydispersity can also vary within a wide range. Copolymers derived from two or more monomers and with a different crosslinking degree can also be used as a support. The morphology of the polymer can vary from spheres to a film arrangement on a surface. Thus, in the case of polystyrenes, these can contain a different density of amino groups for anchoring and a variable proportion of styrene or divinylbenzene. The latter also allows crosslinking the polymer, increasing its mechanical strength and decreasing its solubility.

In the case of phosphazenes, two advantageous configurations are of the type shown below:

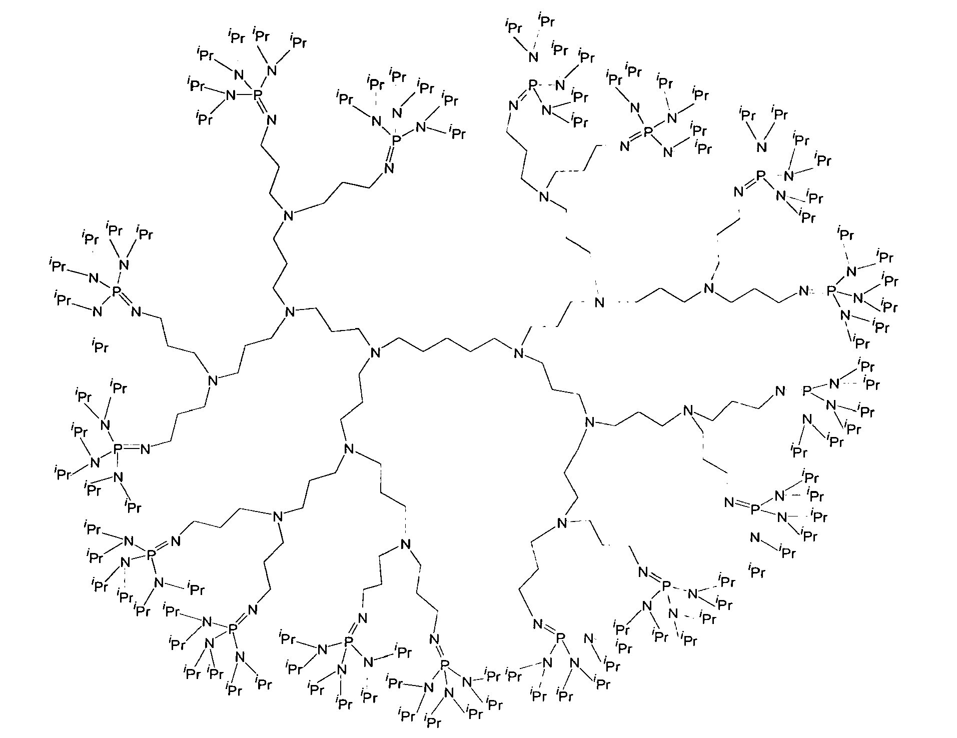

Dendrimer decorated with phosphazene 1 units.

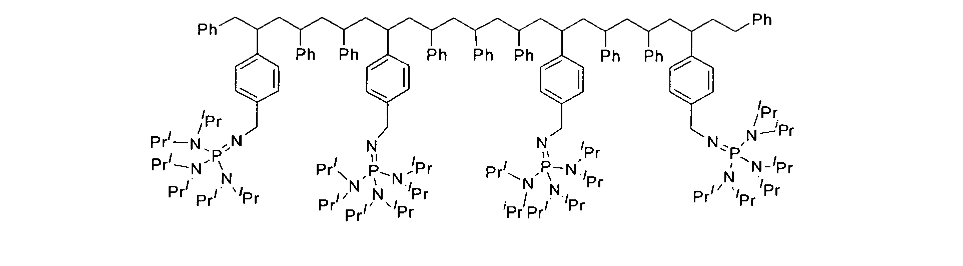

Styrene and 4-aminomethylstyrene copolymer containing phosphazene 1 units.

The solid support can be covalently bonded through at least one connector to the rest of the catalyst, i.e., a group covalently connecting the surface of the insoluble material to the rest of the catalyst and which can have any length and contains different heteroatoms or functional groups.

In the case of phosphazenes, the bonding between the phosphazene and the surface can be carried out through one of the amines, through the imino group or even directly in the phosphorus atom.

### Synthesis of Isocyanates

In addition, it is known that by means of thermolysis, carbamates can undergo alcohol elimination reactions to give isocyanates. This reaction generally requires the presence of catalysts. However, the fluorinated carbamates object of the invention can easily give isocyanates by eliminating the corresponding fluorinated alcohols, even in the absence of catalysts. They are therefore especially suitable for the synthesis of mono- and polyisocyanates.

The methods for transforming fluorinated carbamates into their corresponding isocyanates are known by the person skilled in the art. See WO 98/54128, for example.

For example, the reaction between an amine of formula R₁₄NH₂ with a fluorinated carbonate of formula (OR)(OR')C=O, wherein R₁₄, R and R' are as defined above, according to the process of the present invention, produces a fluorinated carbamate of formula (R₁₄NH)(OR)C=O, for example, which can be transformed into an isocyanate of formula R₁₄-N=C=O by means of the aforementioned methods, thermolysis, for example.

### Definitions

"Alkyl" relates to a linear or branched hydrocarbon chain radical consisting of carbon and hydrogen atoms, which does not contain unsaturation, having between 1 and 20 carbon atoms, preferably 1 to 12, preferably from 1 to 8 carbon atoms, and which is bonded to the rest of the molecule by means of a single bond, methyl, ethyl, n-propyl, i-propyl, n-butyl, t-butyl or n-pentyl, for example.

"Alkylidene" relates to a linear or branched hydrocarbon chain radical consisting of carbon and hydrogen atoms, which does not contain unsaturation, having between 1 and 20 carbon atoms, preferably 1 to 12, preferably from 1 to 8 carbon atoms, and which is bonded to the rest of the molecule by means of two different single bonds, methylidene, ethylidene, 1,3-propylidene, 1,4-butylidene, or 2,4-pentylidene, for example.

"Cycloalkyl" preferably means a linear or branched, monocyclic saturated alicyclic hydrocarbon fraction, having between 3 and 20 carbon atoms, preferably 3 to 12, preferably from 3 to 8 carbon atoms, such as cyclopropyl, cyclobutyl, cyclopentyl, cyclohexyl, cycloheptyl, methyl cyclohexyl, dimethyl cyclohexyl and the like.

"Alkenyl" preferably means a linear or branched, unsaturated monovalent aliphatic hydrocarbon fraction containing 1, 2 or 3 conjugated or unconjugated carbon-carbon double bonds, having between 2 and 20 carbon atoms, preferably 2 to 12, preferably from 2 to 8 carbon atoms, such as -CH=CH₂, -CH₂CH=CH₂, -CH=CH-CH₂, -C(CH₃)=CH₂, -CH=CH-CH=CH₂, and the like;

"Cycloalkenyl" preferably means a monocyclic non-aromatic alicyclic hydrocarbon fraction containing 1 or 2 conjugated or unconjugated carbon-carbon double bonds, having between 4 and 20 carbon atoms, preferably 4 to 12, preferably from 4 to 8 carbon atoms, linear or branched, such as cyclopentenyl, methyl cyclopentenyl, cyclopentadienyl, cyclohexenyl, and the like.

"Alkynyl" preferably means a linear or branched, unsaturated monovalent aliphatic hydrocarbon fraction, containing 1, 2 or 3 conjugated or unconjugated carbon-carbon triple bonds, having between 2 and 20 carbon atoms, preferably 2 to 12, preferably from 2 to 8 carbon atoms, such as -CCH, - CH₂CCH, -CCCH₃, -CH₂CCCH₃, and the like.

"Cycloalkynyl" preferably means a monocyclic non-aromatic alicyclic hydrocarbon fraction containing a 1 or 2 conjugated or unconjugated carbon-carbon triple bonds, having between 7 and 20 carbon atoms, preferably 7 to 12, preferably with 7 or 8 carbon atoms, linear or branched, such as cycloheptenyl, methyl cycloheptenyl, cyclooctinyl, and the like.

"Aryl" preferably means a polycyclic or monocyclic hydrocarbon fraction, having between 6 and 15 carbon atoms, preferably 6 to 12, preferably from 6 to 8 carbon atoms, comprising 1, 2, 3 or 4 aromatic nuclei, said nuclei being bonded with, and/or covalently bonded to one another, such as phenyl, biphenyl, terpenyl, naphthyl, anthracenyl, phenanthrenyl, fluorophenyl, tolyl, trifluoromethyl pentyl, anisyl, indenyl, 2,5-dimethoxy-phenyl and the like;

"Arylalkyl" relates to an aryl group bonded to an alkyl group, having between 7 and 15 carbon atoms, preferably 7 to 12, preferably with 7 or 8 carbon atoms, the alkyl group being bonded to the rest of the molecule, such as benzyl and phenetyl.

"Alkylaryl" relates to an alkyl group bonded to an aryl group, having between 7 and 15 carbon atoms, preferably 7 to 12, preferably with 7 or 8 carbon atoms, the aryl group being joined to the rest of the molecule.

The references of this document to substituted groups in the compounds of the present invention relate to the specified moiety which can be substituted in one or more available positions by one or more suitable groups, for example, a C₁-C₆ alkanoyl group such as acyl and the like; alkyl groups including those groups having from 1 to 12 carbon atoms, more preferably 1-3 carbon atoms; alkenyl and alkynyl groups; alkoxyl groups. Unless otherwise indicated, an optionally substituted group can have a substituent in each position of the group which can be substituted, and each substitution is independent of the other one.

### EXAMPLES

Non-limiting examples of the present invention will be described below.

### i) Obtaining heterogeneous catalysts for the amine carbamoylation reaction.

### Example 1: Preparation of 1,5,7-triazabicyclo[4.4.0]-5-decene covalently anchored to mesoporous silica.

Silica functionalized with 3-choropropyl groups is previously prepared by reacting 5 g of mesoporous silica MCM-41 (previously dehydrated by treatment under vacuum of 10⁻² Torr and a temperature of 150°C for 5 h) with 1 g of 3-chloropropyltriethoxysilane in 20 ml of toluene at reflux temperature and in a nitrogen atmosphere and with magnetic stirring for 5 h. After this time, the solid is collected by filtration, washed abundantly with toluene and dried in an oven at 70°C. An amount of this material containing chlorine groups (5 g) is then treated with a solution of 0.5 g of 1,5,7-triazabicyclo[4.4.0]-5-decene in 20 ml of toluene, the suspension being magnetically stirred at reflux temperature in an inert nitrogen atmosphere for 24 h. After this time, solid is collected by filtration and washed with toluene (3×20 ml) and dichloromethane (3×20 ml). The solid is allowed to dry at room temperature. Scheme 3 summarizes the sequence which is followed to prepare triazabicyclodecene anchored to silica. The resulting solid can optionally be subjected to a subsequent silylation reaction with an excess of propyltrimethoxysilane for the purpose of minimizing the presence of free silanol groups on the surface of the solid.

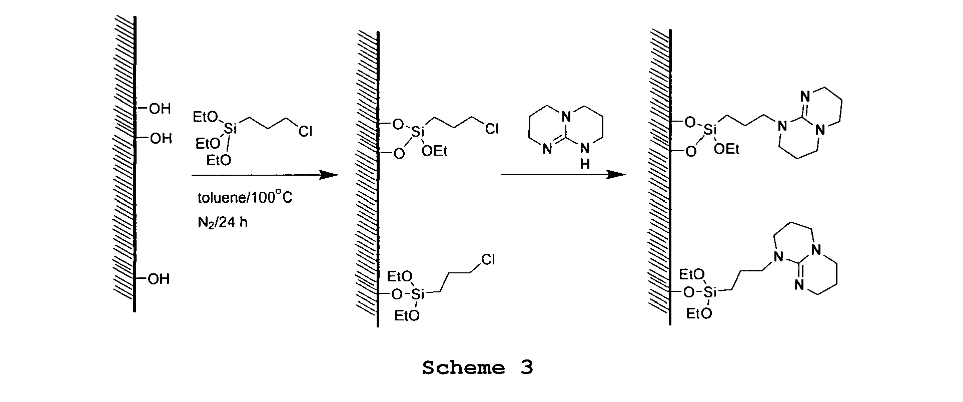

The same material can also be alternatively prepared by reacting in a prior step 1,5,7-triazabicyclo[4.4.0]-5-decene with 3-chloropropyltriethoxysilane and as a last step, carrying out the covalent bonding of the bicycle functionalized with a terminal triethoxysilane group with the surface of the solid. In this case, equimolar amounts of 1,5,7-triazabicyclo[4.4.0]-5-decene (1.0 g) and 3-chloropropyltriethoxysilane (1 equivalent) are reacted at room temperature and in an inert nitrogen atmosphere with constant magnetic stirring for 24 h. After this time, the resulting crude product is used in the following step without carrying out any purification. The reaction is carried out as described above but using MCM-41 (1.0 g) and the bicycle functionalized with triethoxysilyl. Scheme 4 shows this alternative route. The same processes are useful when another type of silicas and inorganic supports, including amorphous silicas, alumina-silicas and metal oxides, are used instead of MCM-41 type mesoporous silica.

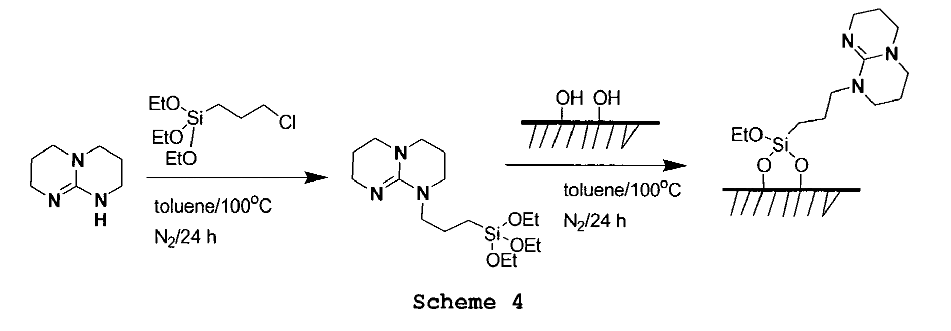

### Example 2: Preparation of a polymer containing N,N-dialkylpyridine substructures.

N-methylaminopyridine (1.0 g) is dissolved in 20 ml of previously dried tetrahydrofuran (THF) and the resulting solution is added dropwise to a suspension of sodium hydride (0.35 g) in dry THF (10 ml). The resulting mixture is stirred for 2 h at room temperature. After this time, a solution of 3-chloropropene (2.22 ml) dissolved in dry THF (2 ml) is added dropwise at 0°C with vigorous stirring. After the addition, the reaction mixture is heated to the reflux temperature for 8 h. The crude product of the reaction is purified by column chromatography using mixtures of hexanes/dichloromethane as an eluent.

N-allyl-N-methyl-4-aminopyridine is used together with styrene (and optionally *para*-divinylbenzene) in the formation of a copolymer. A mixture of styrene and (N-allyl-N-methylamino)pyridine in a molar 20:80 ratio dissolved in 1-octanol (3 ml) is thus purged with a stream of dry nitrogen for 30 min. After this time, 200 mg of azo-bis(isobutyronitrile) is added and the solution is stirred at 80°C for 3 h. The resulting polymer is washed with tetrahydrofuran (3×10 ml) and dried. Scheme 5 shows the route which is followed in the preparation of a polymer containing *N,N*-dialkylaminopyridine.

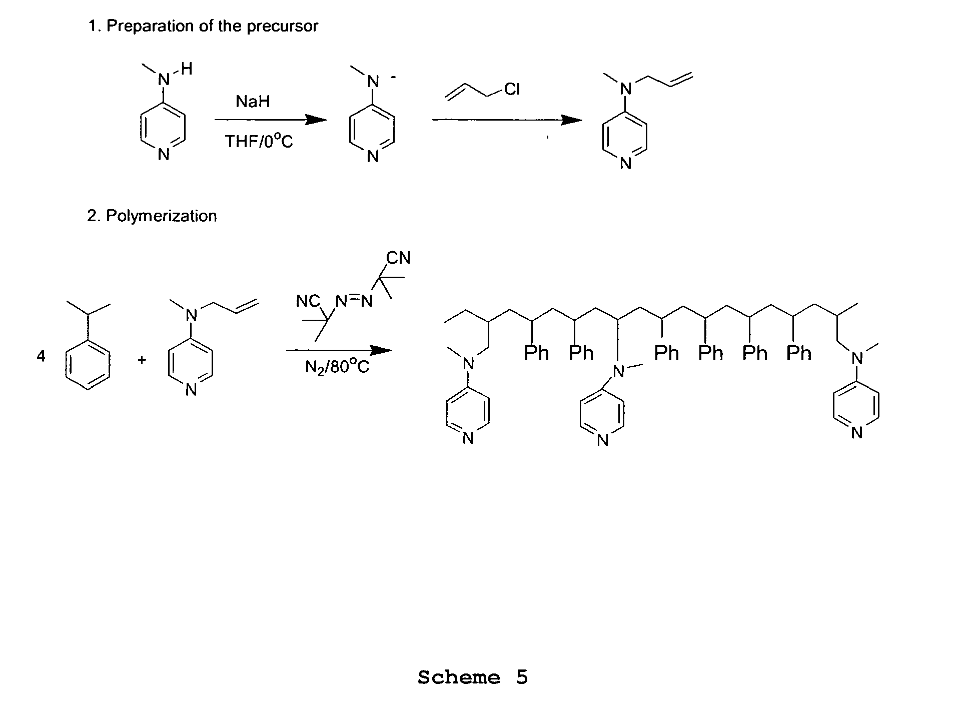

### Example 3. Preparation of the phosphazene anchored to the second generation polyiminoamine dendrimer.

8 equivalents of sodium hydride are added to a solution in dry toluene (10 ml) of a second generation polypropylimino dendrimer (1.0 g) containing eight peripheral amino groups and the suspension is stirred for 30 minutes at -5°C. After this time, eight equivalents of anhydrous phosphorus pentachloride are carefully added with vigorous stirring while the reaction temperature is controlled at -5°C. Once the addition is completed, the mixture is kept stirring for another 30 minutes. Then, keeping the temperature at -5°C, 24 equivalents of lithium diisopropylamide are added and the suspension is stirred for 60 min. The suspension is finally heated at 50°C and the mixture is kept reacting for 3 h. Once the reaction has ended, the suspension is filtered in a dry atmosphere and concentrated under vacuum. The residue is used as a homogenous catalyst for amine carbamoylation.

### ii) Amine carbamoylation with polyfluorinated carbonates.

### Example 4. Process for preparing 2,4-bis(2,2,2-trifluoroethoxycarbonylamino)toluene by reacting 2,4-toluenediamine with bistrifluoroethyl carbonate using phosphazene 2 as a catalyst.

6 mol% of phosphazene-2: 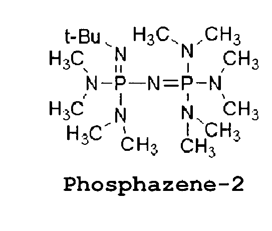 is added to a solution of 2,4-toluenediamine (1.21 g) in 10 ml of bistrifluoroethyl carbonate and the mixture is placed in an autoclave and is heated for 20 h at a temperature of 140°C. After this time, the autoclave is allowed to cool to room temperature and the 2,4-bis(2,2,2-trifluoroethoxycarbonylamino)toluene product is obtained with an 80% yield as a white solid after concentrating the crude product of the reaction. The compound is purified by recrystallizing trifluoroethanol. Scheme 6 shows the process for the dicarbamoylation of 2,4-toluenediamine.

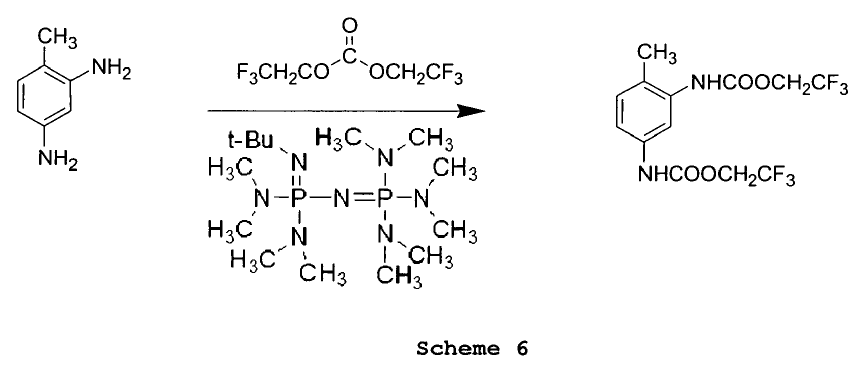

For the purposes of establishing a comparison, two control tests are carried out. One of them was carried out in the same conditions as those indicated above (1.21 g of 2,4-toluenediamine dissolved in 10 ml of bistrifluoroethyl carbonate at 140°C for 20 h) but without adding phosphazene as a catalyst. The formation of the corresponding dicarbamate, 2,4-bis(2,2,2-trifluoroethoxycarbonylamino)toluene was not observed, not even in small amounts, in these conditions in the absence of phosphazene.

The second test was carried out in a manner similar to that indicated above (1.21 g of 2,4-toluenediamine in the presence del 6 mol% of phosphazene-2 and dissolved in 10 ml of carbonate, carrying out the reaction at 140°C for 20 h), but substituting the fluorinated carbonate with dimethyl carbonate. In this case, a product distribution was obtained in which 2,4-bis(methoxycarbonylamino)toluene was not present, but the two products the structures of which are shown below in the indicated percentages were. This product distribution in which carbamoylation and *N*-methylation occur simultaneously in the conditions shows the advantage of working with polyfluorinated carbonate.

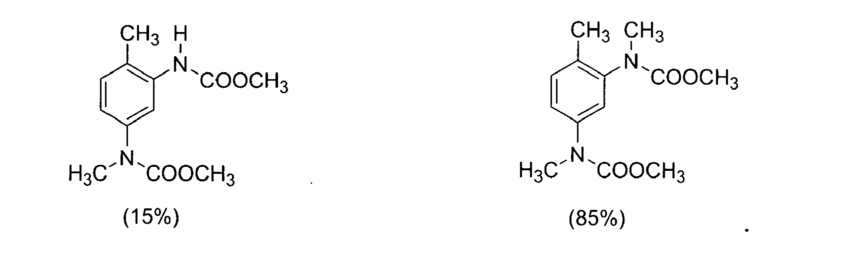

### Example 5. Reaction of cyclohexylamine with bistrifluoroethyl carbonate catalyzed by poly(styrene-co-N-allyl-N-methyl-4-aminopyridine).

The polymer the preparation of which was described in Example 2 can be used as a catalyst for the carbamoylation of cyclohexylamine. A mixture of cyclohexylamine (1.0 g) is dissolved in 10 ml of bistrifluoroethyl carbonate. The solution is placed in an autoclave reactor with a 50 ml capacity and 200 mg of the polymeric catalyst are added. The mixture is heated at 140°C with constant mechanical stirring for 5 h. After this time, the reactor is allowed to cool, the liquid is evaporated under reduced pressure, and the residue is purified by column chromatography, N-(2,2,2-trifluoroethylcarbonylamino)cyclohexane being obtained with a 90% yield.

### Example 6. Reaction of 2,4-toluenediamine with bistrifluoroethyl carbonate catalyzed by 4,4-dimethyl-aminopyridine).

A mixture of 2,4-toluenediamine (1 g) is dissolved in 10 ml of bistrifluoroethyl carbonate. The solution is placed in an autoclave reactor with a 50 ml capacity and 0.1 g of 4,4-dimethylaminopyridine are added. The mixture is heated at 140°C with constant mechanical stirring for 48 hours. After this time, the reactor is allowed to cool, the liquid is evaporated under reduced pressure, and the residue is purified by column chromatography, 2,4-bis-(trifluoroethoxycarbonylamino)toluene being obtained with a 90% yield.

## Claims

1. A process for producing fluorinated carbamates comprising the reaction between an amine or polyamine and a fluorinated carbonate of formula (OR)(OR')C=O, wherein R and R' are independently selected from a substituted or non-substituted fluorinated alkyl group with 1 to 20 carbon atoms, in the presence of a catalyst which does not contain metals and comprises at least one tertiary amine group.

2. A process according to claim 1, wherein the pka of the amine group of the catalyst is greater than 6, preferably greater than 9, preferably a pka greater than 12, more preferably a pka greater than 14.

3. A process according to any of the previous claims, wherein the catalyst is selected from the group consisting of
- a phosphazene;
- a cyclic amine of formula (IV) 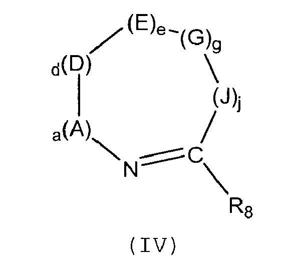 wherein each of a, d, e, g and j is 0 or 1, the total sum of a, d, e, g and j being equal to 3, 4 or 5;
each of the ring members A, D, E, G and J is independently selected from the group consisting of - C(H)=, -C(R₇)=, -N=, -N(R₇)-, -S-, and -O-, the members -N=, -N(R₇)-, -S-, and -O- not being able to be in positions contiguous to one another and the sum of -N=, -N(R₇)-, -S-, and -O- being equal to 0, 1 or 2;
wherein R₇ is selected from the group consisting of substituted or non-substituted alkyl with 1 to 20 carbon atoms and dialkylamino of formula -N(R₉)(R₁₀), wherein R₉ and R₁₀ are independently selected from a substituted or non-substituted alkyl group with 1 to 20 carbon atoms; and
R₈ is selected from the group consisting of hydrogen, substituted or non-substituted alkyl with 1 to 20 carbon atoms and a dialkylamino group of formula - N(R₉)(R₁₀), wherein R₉ and R₁₀ are independently selected from a substituted or non-substituted alkyl group with 1 to 20 carbon atoms; and
- an amine of formula (V) 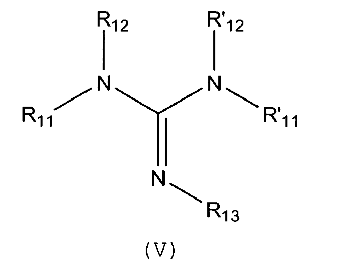 wherein each R₁₁, R₁₂, R₁₃, R'₁₁ and R'₁₂ group is independently selected from the group consisting of substituted or non-substituted alkyl with 1 to 20 carbon atoms and substituted or non-substituted cycloalkyl with 3 to 20 carbon atoms; and wherein at least one R₁₁, R₁₂, R₁₃, R'₁₁ or R'₁₂ group can form together with another R₁₁, R₁₂, R₁₃, R'₁₁ or R'₁₂ group a substituted or non-substituted alkylidene group with 1 to 20 carbon atoms.

4. A process according to claim 3, wherein the catalyst is a phosphazene of formula (I) 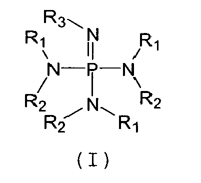 wherein
each R₁ and R₂ is independently selected from the group consisting of a substituted or non-substituted alkyl group with 1 to 20 carbon atoms, a substituted or non-substituted aryl group with 6 to 15 carbon atoms, a substituted or non-substituted arylalkyl group with 7 to 15 carbon atoms and a substituted or non-substituted alkylaryl group with 7 to 15 carbon atoms;
and wherein at least one R₁ or R₂ group can form together with another R₁ or R₂ group a substituted or non-substituted alkylidene group with 1 to 20 carbon atoms; or R₁ and R₂ together form a group of formula (II) 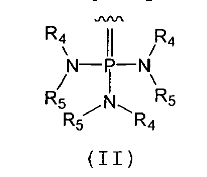 wherein each R₄ and R₅ is independently selected from the group consisting of a substituted or non-substituted alkyl group with 1 to 20 carbon atoms, a substituted or non-substituted aryl group with 6 to 15 carbon atoms, a substituted or non-substituted arylalkyl group with 7 to 15 carbon atoms and a substituted or non-substituted alkylaryl group with 7 to 15 carbon atoms;
and wherein at least one R₄ or R₅ group can form together with another R₄ or R₅ group a substituted or non-substituted alkylidene group with 1 to 20 carbon atoms; or R₄ and R₅ together form a group of formula (II); and
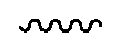 indicates where the group of formula (II) is bonded to the rest of the phosphazene; and
R₃ is selected from the group consisting of a substituted or non-substituted alkyl group with 1 to 20 carbon atoms, a substituted or non-substituted aryl group with 6 to 15 carbon atoms, a substituted or non-substituted arylalkyl group with 7 to 15 carbon atoms and an alkylaryl group with 7 to 15 carbon atoms;
the total number of phosphorus atoms being comprised between 1 and 40
and salts thereof.

5. A process according to claim 4, wherein said phosphazene is selected from the group consisting of

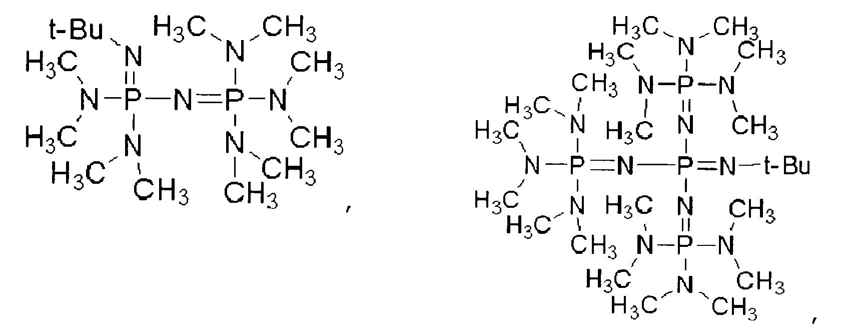

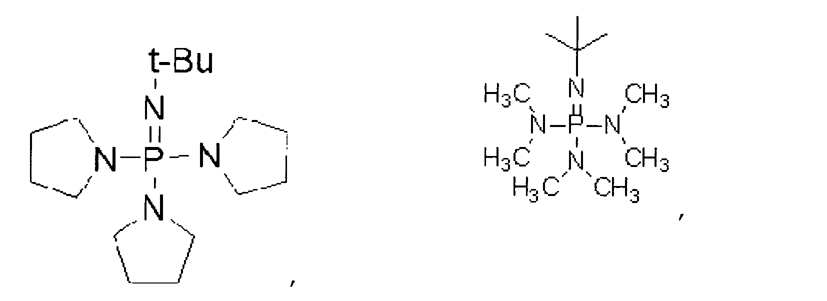

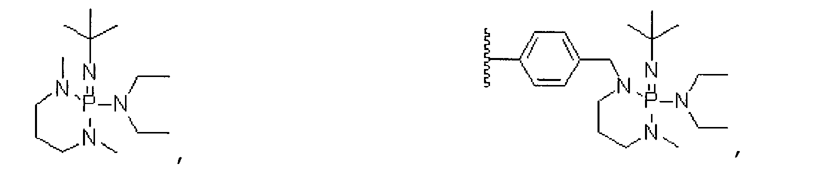

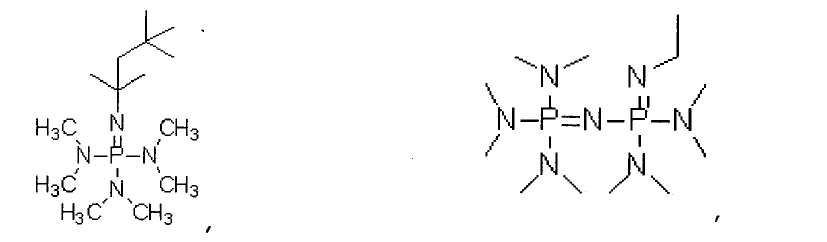

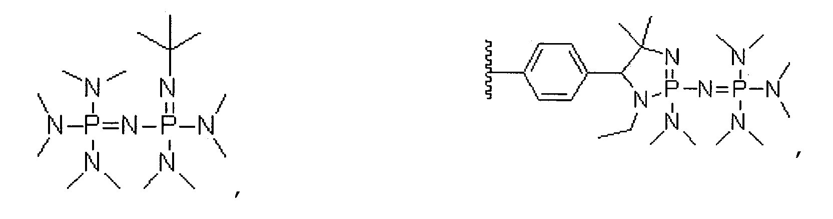

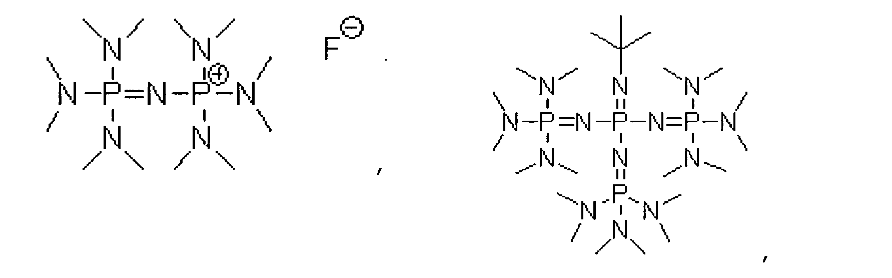

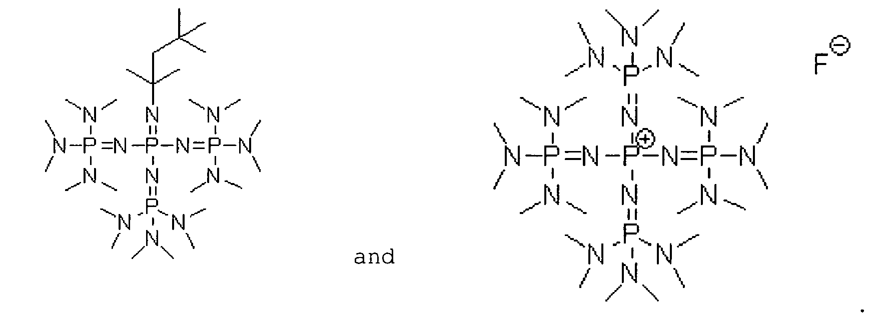

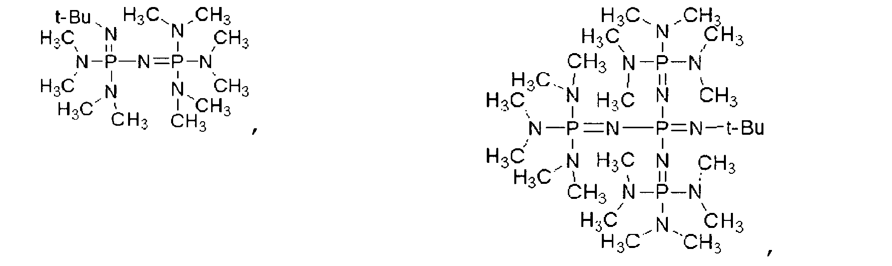

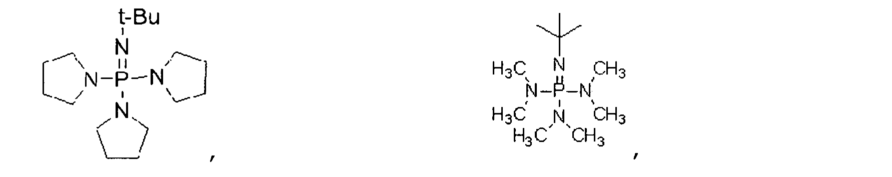

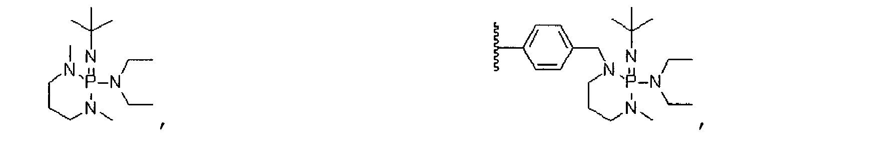

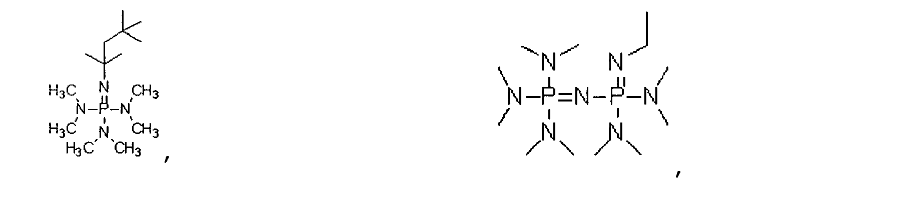

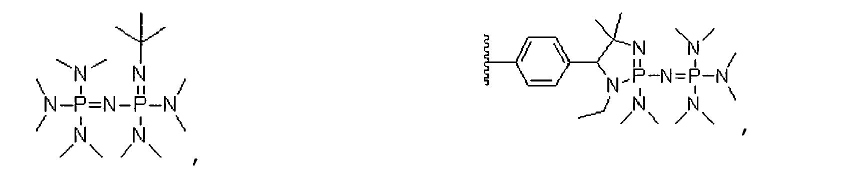

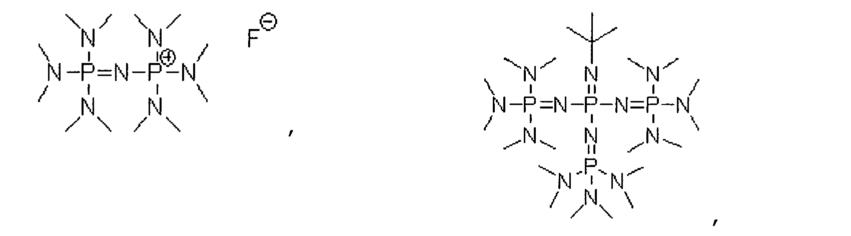

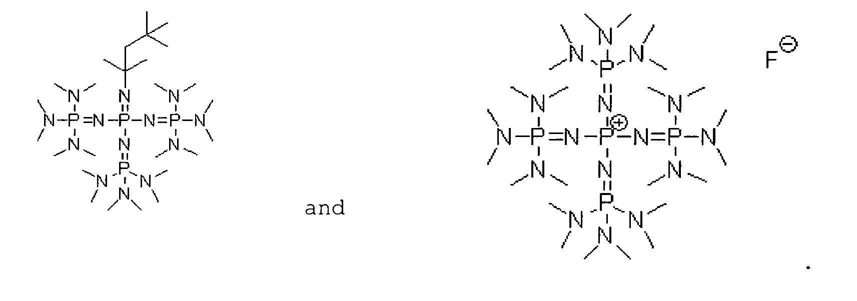

6. A process according to claim 3, wherein the cyclic amine of formula (IV) is a pyridine of formula (IV') 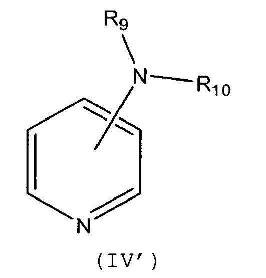 wherein R₉ and R₁₀ are defined as in claim 3.

7. A process according to claim 6 wherein R₉ and R₁₀ are methyl.

8. A process according to claim 3 wherein the catalyst is selected from the group consisting of imidazole, thiazole, 1,5,7-triazabicyclo[4.4.0]-5-decene, 1,6,8-triazabicyclo[5.4.0]-6-undecene and 2,11-diazabicyclo[5.4.0]-1-undecene.

9. A process according to any of the previous claims, wherein said catalyst comprises a solid support.

10. A process according to claim 9, wherein the support is an organic polymer.

11. A process according to claim 10, wherein the support is selected from the group consisting of dendrimers, linear or crosslinked polystyrenes and polyethylene glycol.

12. A process according to claim 9, wherein the support is an inorganic support which is selected from the group consisting of amorphous silicas, structured mesoporous silicas such as MCM-41, MCM-48, SBA-15, FDU, mixed silicon and alumina oxides, laminar or fibrous clays, crystalline or amorphous aluminosilicates and zeolites, metal oxides wherein the particle size is submicrometric such as cerium oxide and other rare earth oxides, iron oxide, hydrotalcites, mixed metal oxides and magnesium oxides.

13. A process according to any of claims 9 to 12, wherein said support is covalently bonded through at least one connector to the rest of the catalyst.

14. A process according to any of the previous claims, wherein the carbonate is selected from the group consisting of bis(2-fluoroethyl) carbonate, bis(3-fluoropropyl) carbonate, bis(2,2,2-trifluoroethyl) carbonate, bis(1,3-difluoro-2-propyl) carbonate, bis(1,1,1-trifluoro-2-propyl) carbonate, bis(2,2,3,3-tetrafluoropropyl) carbonate, bis(2,2,3,3,3-pentafluoropropyl) carbonate, bis(1-fluoro-2-butyl) carbonate, bis(2-fluoro-1-butyl) carbonate, bis(1-fluoro-2-methyl-2-propyl) carbonate, bis(2-fluoro-2-methyl-1-propyl) carbonate, bis(1H,1H,2H,2H-perfluoro-1-hexyl) carbonate, bis(perfluorooctyl) carbonate, bis(1,1,1,3,3,3-hexafluoro-2-propyl) carbonate, preferably bis(2,2,2-trifluoroethyl) carbonate.

15. A process according to any of the previous claims, wherein the amine or polyamine is an amine of formula (III)
R₁₄(NH₂)ₙ (III)
wherein R₁₄ is selected from the group consisting of substituted or non-substituted alkyl with 1 to 20 carbon atoms, substituted or non-substituted aryl with 6 to 15 carbon atoms, substituted or non-substituted arylalkyl with 7 to 15 carbon atoms, substituted or non-substituted alkenyl with 2 to 20 carbon atoms, substituted or non-substituted alkynyl with 2 to 20 carbon atoms, substituted or non-substituted cycloalkyl with 3 to 20 carbon atoms, substituted or non-substituted cycloalkenyl with 4 to 20 carbon atoms and substituted or non-substituted cycloalkynyl with 7 to 20 carbon atoms; and
n is 1, 2, 3, 4, 5 or 6.

16. A process according to any of the previous claims wherein said amine or polyamine is selected from the group consisting of n-propylamine, isopropylamine, n-butylamine, n-hexylamine, n-octylamine, laurylamine, cyclopentylamine, cyclohexylamine, ciclooctylamine, 1,2-diaminoethane, 1,12-diaminododecane, 1,4-diaminocyclohexane, 1,3-bis(aminomethyl)cyclohexane, 1,4-bis(aminomethyl)cyclohexane, hydrogenated 2,4-diaminodiphenylmethane, hydrogenated toluylenediamine, aniline, benzylamine, 2-aminotoluene, 4-aminotoluene, 2,4'-diaminodiphenylmethane, 4,4'-diaminodiphenylmethane, 2,2'-diaminodiphenylmethane, 2,4-toluenediamine, 2,6 toluenediamine, m-phenylenediamine, 1,5 diaminonaphthalene and mixtures thereof.

17. A process according to any of the previous claims, wherein the reaction is carried out at temperatures between 100 and 250°C and sufficient pressure so as to maintain the reactants in liquid phase.

18. A process according to any of the previous claims, comprising the additional step of transforming the fluorinated carbamates obtained into the corresponding isocyanate.
